# EUROPÄISCHE PATENTSCHRIFT

(11) **EP 3 262 130 B1**
(45) Veröffentlichungstag und Bekanntmachung des Hinweises auf die Patenterteilung: **28.10.2020**
(21) Anmeldenummer: 16706350.2
(22) Anmeldetag: 24.02.2016
(51) Int. Cl.: C09J 4/00

(54) **VERFAHREN ZUR HERSTELLUNG VON ISOSORBIDDI(METH)ACRYLAT**
METHOD FOR PRODUCING ISOSORBIDE DIMETHACRYLATE
PROCÉDÉ DE PRÉPARATION DE DI(MÉTH)ACRYLATE D'ISOSORBIDE

(30) Priorität: 26.02.2015 US 201562120922 P
(43) Veröffentlichungstag der Anmeldung: 03.01.2018
(73) Patentinhaber: BASF SE, 67056 Ludwigshafen am Rhein (DE)
(72) Erfinder: MISSKE, Andrea, 67346 Speyer (DE); FLEISCHHAKER, Friederike, 67065 Ludwigshafen (DE); FLECKENSTEIN, Christoph, 63579 Freigericht-Somborn (DE); KALLER, Martin, 68199 Mannheim (DE); NAIR, Ritesh, 69115 Heidelberg (DE); STENGEL, Ulrik, 69488 Birkenau (DE); BLANCHOT, Mathieu, 67245 Lambsheim (DE)
(86) Internationale Anmeldenummer: PCT/EP2016/053858
(87) Internationale Veröffentlichungsnummer: WO 2016/135191

(56) Entgegenhaltungen:
- US-A- 3 887 609
- US-A- 5 288 767
- US-A1- 2011 130 582
- ZHILA VAZIFEHASL ET AL: "New Series of Dimethacrylate-Based Monomers on Isosorbide as a Dental Material: Synthesis and Characterization", INTERNATIONAL JOURNAL OF COMPOSITE M ATERIALS, Bd. 3, Nr. 4, 1. Januar 2013 (2013-01-01), Seiten 100-107, XP55268012, US ISSN: 2166-479X, DOI: 10.5923/j.cmaterials.20130304.03

## Beschreibung

Die Erfindung gemäß Ansprüchen 1 bis 14 betrifft ein Verfahren zur Herstellung von Isosorbiddi(meth)acrylat durch Umesterung von Alkyl(meth)acrylat mit Isosorbid und die Verwendung von Isosorbiddi(meth)acrylat als Harzkomponent für Zweikomponenten-Klebemasse.

E2BADMA (Dimethacrylat von mit jeweils 2 Ethylenoxid-Einheiten alkoxyliertem Bisphenol-A) und E3BADMA (Dimethacrylat von mit jeweils 3 Ethylenoxid-Einheiten alkoxyliertem Bisphenol-A) sind Bisphenol A-basierte Dimethacrylate, die als Vernetzer zum Beispiel in Coatings und Mischungen für Bohrlöcher zum Einsatz kommen. Sie zeichnen sich durch eine schnelle Reaktionszeit aus, die dazu führt, dass 2-Komponenten-Mischungen schon nach kurzer Zeit teillastfest sind und die Endzugfestigkeit nach wenigen Stunden erreicht ist. Des Weiteren besitzen sie eine hohe Polymerisatdichte mit sehr gutem Kohäsions-/Adhäsions-Verhältnis, eine gute Kristallisationsfestigkeit bei gleichzeitig niedrigem Schrumpf und geringer Versprödungstendenz sowie eine hohe Benetzungskraft, um ein gutes Kriechvermögen in Kapillarrisse zu gewährleisten.

DE 41 31 458 A1 offenbart Zweikomponenten-Klebemassen für die chemische Befestigungstechnik umfassend ein Kunstharz enthaltend das Di(meth)acrylat eines alkoxylierten Bisphenols sowie ein Härtungsmittel für das Kunstharz.

Für bestimmte Anwendungen wird nach Bisphenol A-freien Vernetzern mit ähnlichem Eigenschaftsprofil gesucht.

Isosorbid ist ein Diol auf Basis nachwachsender Rohstoffe und bietet sich strukturell als Alternative zu Bisphenol A an, da es ein ähnlich starres Grundgerüst besitzt.

Eine Möglichkeit, Di(meth)acrylate von Isosorbid herzustellen, ist die Veresterung von Isosorbid mit (Meth)acrylsäure oder die Umesterung von Methylacrylat, Ethylacrylat oder Methylmethacrylat mit Isosorbid in Gegenwart geeigneter Katalysatoren. Beiden OH-Gruppen des Isosorbids sind sekundäre OH-Gruppen mit vergleichsweise geringer Reaktivität, wobei die relativen Reaktivitäten der beiden OH-Gruppen zudem sehr unterschiedlich sind. Somit ist ein unvollständiger Umsatz zu erwarten.

DE 2 317 226 A1 offenbart ein Verfahren zur Herstellung von (Meth)acrylsäureestern aus einem Gemisch von C₁₀-C₁₈-Alkanolen durch Umesterung von Methyl(meth)acrylat in Gegenwart von Titanalkoholat als Katalysator und 2,6-Di-tert.-butylparakresol (TBK) als Stabilisator. Dabei wird in Gegenwart von Aktivkohle gearbeitet. Nach Beendigung der Reaktion wird Wasser zugegeben, wodurch das Titanalkoholat zu Titanhydroxid/-oxid hydrolysiert wird, welches an die Aktivkohle adsorbiert. Der Feststoff wird abfiltriert und das Reaktionsprodukt einer Wasserdampfdestillation unterzogen.

WO 2009/080380 offenbart ein Verfahren zur Herstellung von Methacrylaten von C₆-C₂₂-Alkoholen durch Umesterung von Methyl(meth)acrylat mit den entsprechenden Alkoholen in Gegenwart von Titanalkoholat als Katalysator. In Beispiel 1 wird Methylmethacrylat mit 2-Ethylhexanol in Gegenwart von Hydrochinonmonomethylether (MEHQ) als Stabilisator und Tetraisopropyltitanat als Katalysator umgesetzt. Dabei wird ein azeotropes Gemisch aus Methanol/Methylmethacrylat abdestilliert. Nach Abdestillieren von nicht umgesetztem Methylmethacrylat wird das Katalysator enthaltende 2-Ethylhexylmethacrylat einer Reindestillation im Vakuum (ca. 30 mbar) unterworfen. Dabei wird 2-Ethylhexylmethacrylat mit einer Reinheit von 99,4 % erhalten.

Aufgabe der Erfindung ist es, ein Verfahren zur Herstellung von Isosorbiddi(meth)acrylat durch Umesterung von Alkyl(meth)acrylat mit Isosorbid bereitzustellen, bei dem nur in geringem Umfang Nebenprodukte gebildet werden.

Gelöst wird die Aufgabe durch ein Verfahren zur Herstellung von Isosorbiddi(meth)acrylat durch Umesterung von Alkyl(meth)acrylat mit Isosorbid, umfassend die Schritte:
(i) Reagieren lassen von Alkyl(meth)acrylat mit Isosorbid in Gegenwart eines Titan(IV) oder Zirkonium(IV) enthaltenden Katalysators und eines Stabilisators in Gegenwart eines Schleppmittels, das mit dem in dem Alkyl(meth)acrylat gebundenen Alkohol ein Azeotrop bildet,
(ii) kontinuierliches Abdestillieren des Azeotrops aus Schleppmittel und Alkohol,
   wobei die Schritte (i) und (ii) gleichzeitig durchgeführt werden, bis Isosorbid im Wesentlichen vollständig umgesetzt ist,
(iii) Zugabe von Wasser zu dem in den Schritten (i) und (ii) erhaltenen, Isosorbiddi(meth)acrylat enthaltenden Produktgemisch und Abtrennung von Hydrolysaten des Titan(IV) oder Zirkonium(IV) enthaltenden Katalysators,
(iv) Abdestillieren von nicht umgesetztem Alkyl(meth)acrylat und Schleppmittel aus dem Produktgemisch,
(v) Abdestillieren von Wasser aus dem Produktgemisch,
wobei Schritt (iv) auch vor Schritt (iii) durchgeführt werden kann und die Schritte (iv) und (v) auch in einem Destillationsschritt durchgeführt werden können.

Überraschenderweise wurde gefunden, dass durch Umesterung von Alkyl(meth)acrylat mit Isosorbid in Gegenwart eines Titan(IV) oder Zirkonium(IV) enthaltenden Katalysators Isosorbiddi(meth)acrylat in hohen Ausbeuten gebildet wird.

Durch vollständiges Entfernen des als Koppelprodukt gebildeten Methanols oder Ethanols aus dem Reaktionsgemisch wird das Zielprodukt in hoher Reinheit erhalten.

Der Gehalt an Nebenprodukten an dem nach Schritt (v) erhaltenen Produkt beträgt vorzugsweise < 4 Gew.-%. Nebenprodukte sind vor allem die Isosorbid-Mono(meth)acrylate. Daneben kann das nach Schritt (v) erhaltene Produkt nicht umgesetztes Isosorbid enthalten. Dieses stellt kein Nebenprodukt dar. Im Allgemeinen beträgt der Gehalt des nach Schritt (v) erhaltenen Produktes an Isosorbid bis zu 2 Gew.-%, bevorzugt bis zu 1 Gew.-%. Daneben kann das nach Schritt (v) erhaltene Produkt noch Spuren von Schleppmittel, Alkyl(meth)acrylat und Wasser enthalten. Diese stellen ebenfalls keine Nebenprodukte dar und können in Mengen von insgesamt bis zu 2 Gew.-%, bevorzugt bis zu 1 Gew.-% in dem nach Schritt (v) erhaltenen Produkt enthalten sein.

Die Menge aller Nebenkomponenten (einschließlich Nebenprodukten, Isosorbid, Schleppmittel, Alkyl(meth)acrylat, Wasser) an dem nach Schritt (v) erhaltenen Produkt beträgt im Allgemeinen bis zu 6 Gew.-%, bevorzugt bis zu 4 Gew.-%.

Geeignete Alkyl(meth)acrylate sind die C₁-C₄-Alkyl(meth)acrylate. Im Allgemeinen wird das Methyl(meth)acrylat oder Ethyl(meth)acrylat eingesetzt, wobei bei der Umesterungsreaktion Methanol oder Ethanol als Alkohole freigesetzt werden.

Die Reaktion von Alkyl(meth)acrylat mit Isosorbid erfolgt in Gegenwart eines Titan(IV) oder Zirkonium(IV) enthaltenden Katalysators. Geeignete Titan(IV) oder Zirkonium(IV) enthaltende Katalysatoren sind die Ti(IV)- bzw. Zr(IV)-tetraalkoxylate von linearen oder verzweigten C₁-C₆-Alkoholen, vorzugsweise die Tetraisopropylate, Tetrabutylate sowie das Metallat des eingesetzten Eduktalkohols oder Mischungen hieraus. Auch mit unterschiedlichen Alkoholen oder mit Acetylaceton substituierte Metallate sind möglich.

Die Reaktion von Alkyl(meth)acrylat mit Isosorbid erfolgt weiterhin in Gegenwart eines oder mehrerer Stabilisatoren (Polymerisationsinhibitoren). Geeignete Stabilisatoren können beispielsweise N-Oxide (Nitroxyl- oder N-Oxyl-Radikale, also Verbindungen, die wenigstens eine N-O-Gruppe aufweisen), wie zum Beispiel 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, Bis(1-oxyl-2,2,6,6-tetramethylpiperidine-4-yl)sebacat, 4,4',4"-Tris(2,2,6,6-tetramethyl-piperidin-N-oxyl)-phosphit oder 3-Oxo-2,2,5,5-tetramethylpyrrolidin-N-oxyl; ein- oder mehrwertige Phenole, die ggf. eine oder mehrere Alkylgruppen aufweisen, wie zum Beispiel Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie zum Beispiel Hydrochinon, Hydrochinonmonomethylether, 2-Methylhydrochinon oder 2,5-Di-tert.-Butylhydrochinon; Hydroxyphenole, wie beispielsweise Brenzcatechin (1,2-Dihydroxybenzol) oder Benzochinon; Aminophenole, wie zum Beispiel p-Aminophenol; Nitrosophenole, wie zum Beispiel p-Nitrosophenol; Alkoxyphenole, wie beispielsweise 2-Methoxyphenol (Guajacol, Brenzcatechinmonomethylether), 2-Ethoxyphenol, 2-lsopropoxyphenol, 4-Methoxyphenol (Hydrochinonmonomethylether), Mono- oder Di-tert.-Butyl-4-methoxyphenol; Tocipherole, wie zum Beispiel α-Tocopherol sowie 2,3-Dihydro-2,2-dimethyl-7-hydroxybenzofuran (2,2-Dimethyl-7-hydroxycumaran), aromatische Amine, wie zum Beispiel N,N-Diphenylamin oder N-Nitrosodiphenylamin; Phenylendiamine, wie zum Beispiel N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie zum Beispiel N,N'-Dimethyl-p-phenylendiamin oder N,N'-Diethyl-pphenylendiamin, Hydroxylamine, wie zum Beispiel N,N-Diethylhydroxylamin, Imine, wie zum Beispiel Methylethylimin oder Methylenviolett, Sulfonamide, wie zum Beispiel N-Methyl-4-toluolsulfonamid oder N-tert.-Butyl-4-toluolsulfonamid, Oxime, wie Aldoxime, Ketoxime oder Amidoxime, wie zum Beispiel Diethylketoxim, Methylethylketoxim oder Salicyladoxim, phosphorhaltige Verbindungen, wie zum Beispiel Triphenylphosphin, Triphenylphosphit, Triethylphosphit, hypophsophorige Säure oder Alkylester der Phosphorigen Säuren; schwefelhaltige Verbindungen wie zum Beispiel Diphenylsulfid oder Phenothiazin oder Gemische davon sein.

Bevorzugt sind Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, 2-tert.-Butylphenol, 4-tert.-butylphenol, 2,4-Di-tert.-butylphenol, 2-tert.-Butyl-4-methylphenol, 6-tert.-Butyl-2,4-dimethylphenol, 2,6-Di-tert.-Butyl-4-methylphenol und 2-Methyl-4-tert.-butylphenol und Phenothiazin.

Besonders bevorzugt ist Hydrochinonmonomethylether (MeHQ) und Phenothiazin (PTZ).

Vorteilhaft kann zusätzlich als Polymerisationsinhibitor Sauerstoff eingesetzt werden.

Zur weiteren Stabilisierung kann ein sauerstoffhaltiges Gas, bevorzugt Luft oder ein Gemisch aus Luft und Stickstoff (Magerluft) anwesend sein.

Die Umesterungsreaktion (Schritte (i) und (ii)) wird im Allgemeinen bei einer Temperatur von 60 bis 140°C, bevorzugt 70 bis 110°C durchgeführt. Dabei wird ein Azeotrop aus Schleppmittel und Alkohol kontinuierlich abdestilliert.

Geeignete, mit Methanol oder Ethanol ein azeotrop siedendes Gemisch bildende Schleppmittel sind zunächst Methylacrylat und Methylmethacrylat sowie Ethylacrylat und Ethylmethacrylat selbst. Als separate Schleppmittel geeignet sind unter anderem Cyclohexan, Methylcyclohexan, Benzol, Toluol, Hexane und Heptane und Mischungen hieraus. Bevorzugt sind Methylacrylat, Methylmethacrylat, Ethylacrylat und Ethylmethacrylat sowie Mischungen von diesen mit n-Heptan und Cyclohexan. Der Begriff Schleppmittel umfasst in diesem Sinne das Edukt selbst sowie gegebenenfalls ein zusätzlich eingesetztes separates Lösungsmittel.

In einer bevorzugten Ausführungsform wird kein separates Lösungsmittel als Schleppmittel eingesetzt. In diesem Fall fungiert das Edukt Alkyl(meth)acrylat selbst als Schleppmittel.

Das Schleppmittel kann anschließend wieder im Reaktor ergänzt werden. Hierzu wird das azeotrope Gemisch aus Alkohol und Schleppmittel in einer bevorzugten Ausführungsform über eine geeignete Kolonne abdestilliert, in einem Mischgefäß mit Wasser gerührt und dann in einen Phasenseparator überführt, wobei sich der Alkohol, im Allgemeinen Methanol oder Ethanol, in Wasser löst und sich die organische Phase als obere Schicht abscheidet. Die organische Phase wird vorzugsweise über den Kopf der Kolonne dem Reaktionsgemisch wieder zugeführt und damit bis auf geringe Verluste im Kreislauf geführt. Es kann aber auch alternativ frisches Schleppmittel hinzugeführt werden und eine Aufarbeitung des Schleppmittel-Alkohol-Gemisches in einem separaten Schritt erfolgen oder auf die Ergänzung des Schleppmittels ganz oder teilweise verzichtet werden.

Im Allgemeinen wird Alkyl(meth)acrylat in stöchiometrischem Überschuss eingesetzt. Vorzugsweise beträgt der Überschuss an Methyl(meth)acrylat pro zu veresternder Hydroxylgruppe 5 bis 500 mol-%, besonders bevorzugt 5 bis 200 mol-%, insbesondere 50 bis 100 mol-%.

Der Katalysator wird in einer Konzentration von 0,1-10 mol-%, bezogen auf die Menge an Isosorbid, eingesetzt, bevorzugt in einer Konzentration von 0,1 bis 5 mol-%.

Die Umesterung kann bei Atmosphärendruck, aber auch bei Überdruck oder Unterdruck durchgeführt werden. Im Allgemeinen wird sie bei 300 bis 1000 mbar, bevorzugt bei 800 -1000 mbar (Atmosphärendruck = 1000 mbar) durchgeführt. Die Reaktionszeit beträgt im Allgemeinen 1 h bis 24 Stunden, vorzugsweise 6 bis 18 Stunden. Die Umesterung (Schritte (i) und (ii)) kann kontinuierlich, beispielsweise in einer Rührkesselkaskade, oder diskontinuierlich erfolgen.

Die Reaktion kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor.

Zur Durchmischung des Ansatzes können beliebige Vorrichtungen eingesetzt werden, wie zum Beispiel Rührvorrichtungen. Die Durchmischung kann auch durch Einspeisen eines Gases, vorzugsweise eines Sauerstoff-haltigen Gases erfolgen.

Das Entfernen des gebildeten Alkohols, im Allgemeinen Methanol oder Ethanol, erfolgt kontinuierlich oder schrittweise in an sich bekannter Weise durch azeotrope Destillation in Gegenwart eines Schleppmittels. Zusätzlich kann Methanol auch durch Strippen mit einem Gas entfernt werden.

In einer bevorzugten Ausführungsform wird aus dem in Schritt (ii) abdestillierten Azeotrop aus Schleppmittel und Alkohol der Alkohol durch Waschen mit Wasser abgetrennt und das Schleppmittel in den Reaktionsbehälter zurückgeführt.

Die Schritte (i) und (ii) werden durchgeführt, bis das eingesetzte Isosorbid im Wesentlichen vollständig zum Diester umgesetzt ist. Dies ist der Fall, wenn Isosorbid zu 85 %, bevorzugt zu 90 %, besonders bevorzugt zu 95 % zum Diester umgesetzt ist.

Anschließend werden die Schritte (iii) und (iv) durchgeführt, die auch in umgekehrter Reihenfolge durchgeführt werden können.

In Schritt (iii) wird zu dem das Isosorbiddi(meth)acrylat enthaltenden Produktgemisch Wasser zugegeben, wodurch der Titan(IV) oder Zirkonium(IV) enthaltende Katalysator zu dem entsprechenden Hydroxid hydrolysiert wird. Das schwerlösliche Hydrolysat wird anschließend abgetrennt, zum Beispiel durch Filtration oder Zentrifugation.

Die Filtration kann beispielsweise mit einer Druckfilternutsche durchgeführt werden. Verfahrenstechnisch können für eine Filtration im erfindungsgemäßen Verfahren alle an sich bekannten Filtrationsverfahren und -apparate eingesetzt werden, zum Beispiel solche, die in Ullmann's Encyclopedia of Industrial Chemistry, 7th Ed. 2013 Electronic Release, Kapitel: Filtration, 1. Fundamentals und Filtration 2. Equipment, beschrieben sind. Beispielsweise können dies Kerzenfilter, Filterpressen, Tellerdruckfilter, Beutelfilter oder Trommelfilter sein. Vorzugsweise werden Kerzenfilter oder Tellerdruckfilter eingesetzt. Die Filtration kann mit oder ohne Filterhilfsmittel durchgeführt werden. Geeignete Filterhilfsmittel sind Filterhilfsmittel basierend auf Kieselgur, Perlit und Cellulose.

Geeignete Zentrifugen und auch Separatoren sind dem Experten bekannt. Verfahrenstechnisch können für eine Zentrifugation im erfindungsgemäßen Verfahren alle an sich bekannten Zentrifugationsverfahren und -apparate eingesetzt werden, zum Beispiel solche, die in Ullmann's Encyclopedia of Industrial Chemistry, 7th Ed. 2013 Electronic Release, Kapitel: Centrifuges, Filtering und Centrifuges, Sedimenting, beschrieben sind.

In einer bevorzugten Ausführungsform werden dann anschließend in den Destillationsschritten (iv) und (v) nicht umgesetztes Alkyl(meth)acrylat sowie Wasser aus dem Produktgemisch abdestilliert. Diese Destillation erfolgt im Allgemeinen bei einer Temperatur von 40 bis 100°C, bevorzugt 60 bis 80°C, und einem variablen Druck von 2 bis 700 mbar. Zusätzlich können diese Komponenten auch durch Strippen mit einem Gas, vorzugsweise einem sauerstoffhaltigen Gas entfernt werden.

Wird kein separates Schleppmittel eingesetzt, so werden die Schritte (iv) und (v) bevorzugt in einem gemeinsamen Destillationsschritt durchgeführt. Wird ein separates Schleppmittel eingesetzt, so wird Schritt (iv) bevorzugt vor Schritt (iii) durchgeführt.

Die destillative Abtrennung erfolgt beispielsweise in einem Rührkessel mit Doppelwandheizung und/oder innenliegenden Heizschlangen unter vermindertem Druck.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 bis 700 mbar, bevorzugt 30 bis 500, besonders bevorzugt 50 bis 150 mbar und einer Temperatur von 40 bis 80°C durch den Apparat geführt.

Vorteilhaft kann ein sauerstoffhaltiges Gas, bevorzugt Luft oder ein Gemisch aus Luft und Stickstoff (Magerluft) in den Destillationsapparat eingeleitet werden, beispielsweise 0,1 bis 1, bevorzugt 0,2 bis 0,8 und besonders bevorzugt 0,3 bis 0,7 m³/m³h, bezogen auf das Volumen des Reaktionsgemisches.

Nach Durchführung der Schritte (iii), (iv) und (v) verbleibt ein Produkt als Sumpfprodukt, welches die oben beschriebene Reinheit aufweist.

Gegenstand der Erfindung ist auch die Verwendung von Isosorbiddi(meth)acrylat als Harzkomponente für Zweikomponenten-Klebemassen.

Erfindungsgemäße Zweikomponentenklebmassen für die chemische Befestigungstechnik enthalten
I. ein Kunstharz mit einer Viskosität bei 23°C zwischen 100 und 10 000 (mPa•s), welches das Isosorbiddi(meth)acrylat enthält, und
II. ein Härtungsmittel für das Kunstharz.

Die eine Komponente der erfindungsgemäßen Klebmasse ist ein Kunstharz mit einer Viskosität (bei 23°C) zwischen 100 und 10 000, vorzugsweise 200 bis 2000 und insbesondere 500 bis 1500 mPa•s, gemessen in Abwesenheit von Füllstoffen. Es enthält das Isosorbiddi(meth)arylat.

Das Kunstharz kann 2 bis 20 Gew.-% anderer härtbarer Harze, wie Polyester-, Vinylester-, Bismaleinimid- oder Epoxid-Harze, sowie zum Zweck der Zähmodifizierung 2 bis 20 Gew.-% eines Thermoplasten, wie Polyamid oder Polyester, oder eines Kautschuks enthalten.

Falls für die Peroxidhärtung Beschleuniger erforderlich sind, werden diese zweckmäßigerweise räumlich zusammen mit dem Harz, d.h. getrennt vom Härter, angeordnet. Geeignete Beschleuniger sind: Aromatische Amine, wie N,N-Dimethylanilin, N,N-Diethylanilin; Toluidine und Xylidine wie N,N-Diisopropyliden-para-toluidin, N,N-Dimethyl-p-toluidin, N,N- Bis-(2-hydroxyethyl)-xylidin; ferner Co-, Mn-, Sn- oder Ce-Salze, wie zum Beispiel Cobaltnaphthenat, sowie Mischungen von Amin- und Cobaltbeschleunigern. Die Beschleuniger sind im Kunstharz im Allgemeinen in Mengen von vorzugsweise 0,5 bis 5 Gew.-% enthalten.

In der Regel wird die Konfektionsform einer 2-Kammerpatrone gewählt. Als Kartuschen werden vorzugsweise Zweikammerkartuschen verwendet, deren größere Kammer das Harz und die kleinere Kammer das Härtungsmittel enthält. Die größere Kammer hat ein etwa 5 bis 10 mal größeres Volumen als die kleinere Kammer.

In der Kammer, welche die Kunstharzkomponente enthält, können daneben auch noch Füllstoffe vorhanden sein. Als verstärkende Füllstoffe für die Klebmasse dienen zum Beispiel Quarz, Glas, Korund, Porzellan, Steingut, Schwerspat, Leichtspat, Talkum und Kreide. Die Füllstoffe werden in Form von Sanden, Mehlen oder speziellen Formkörpern (Zylinder, Kugeln usw.) entweder der Harzlösung und/oder dem Härter (Initiator) zugemischt. Die Füllstoffe können als Fasern (fibrilläre Füllstoffe) eingesetzt werden. Bevorzugt und deutlicher verstärkend wirken sich die globulären, inerten Stoffe (Kugelform) aus.

Räumlich getrennt vom Harz ist das Härtungsmittel vorgesehen. Bevorzugte Härtungsmittel sind bei niedrigen Temperaturen zerfallende organische Peroxide. Besonders gut geeignet sind Benzoylperoxid und Methylethylketonperoxid, ferner tert.-Butylperbenzoat, Cyclohexanonperoxid, Laurylperoxid und Cumolhydroperoxid, sowie Mischungen verschiedener Peroxide. Die Peroxide werden vorzugsweise in Mengen von 0,5 bis 10 Gew.%, vorzugsweise von 1 bis 5 Gew.-% eingesetzt. Die Härtungsmittel werden zweckmäßigerweise auf inerte Füllstoffe aufgebracht, wobei Quarzsande mit Korngrößen von 0,5 bis 3 mm oder von 3 bis 6 mm bei Dimensionen bevorzugt sind.

Im Fall schaumfähiger Klebemassen wird zweckmäßigerweise Carbonat zum Harz gegeben, die Säure-Komponente kann entweder mit dem Härter zusammen in eine Kammer oder aber in eine separate dritte Kammer gefüllt werden.

Die erfindungsgemäße Zweikomponenten-Klebmasse kann als Dübelmasse zur Befestigung von Verankerungen in Bohrlöchern eingesetzt werden. Derartige Verankerungen weisen ein gutes Rissdehnungsverhalten, geringe Schrumpfspannung und ausgezeichnete Adhäsion an mineralischen Aufnahmewerkstoffen, wie Beton und Naturstein, sowie an Schaum- und Hohlblocksteinen auf.

Die Erfindung wird durch die nachstehenden Beispiele näher ausgeführt.

### Beispiele

### Beispiel 1

In einem 0,75L-Planschliffreaktor mit Kolonne, Kühler, Flüssigkeitsteiler, Ankerrührer sowie Lufteinleitung werden Ethylacrylat (626 g), MeHQ (0,31 g), Phenothiazin (0,31 g) sowie Isosorbid (186 g) vorgelegt und unter Lufteinleitung und Rühren auf eine Sumpftemperatur von 68°C hochgeheizt. Bei einem Druck von 300 mbar werden 200 g Ethylacrylat abdestilliert. Titantetraisopropoxylat (7,1 g) wird zudosiert und weiter auf 100°C Sumpftemperatur aufgeheizt. Die Reaktionsmischung ist trüb. Ethylacrylat und gebildetes Ethanol werden mit vollem Ablauf abdestilliert. EA wird portionsweise zudosiert in den Mengen, die dem EA im Destillat entsprechen. Im Verlauf der Reaktion werden weitere 2,9 g Titantetraisopropoxylat zudosiert. Die Sumpftemperatur steigt im Verlauf der Reaktion auf 105°C an. In regelmäßigen Abständen werden Sumpf- und Destillatproben gezogen, um den Verlauf der Reaktion zu beobachten. Es wird ein Vakuum von maximal 810 mbar angelegt, um die Reaktion dann nach 18 h Laufzeit zu komplettieren. Das Reaktionsgemisch wird mit 125 mL Wasser versetzt, über einen Papierfilter abfiltriert und im Vakuum konzentriert. Es wird folgende Zusammensetzung erhalten (GC-FI%): Isosorbid 0 %, Summe der Isosorbid-Monoacrylate 3,3 %, Zielprodukt Isosorbiddiacrylat
96,7 %.

### Vergleichsbeispiel 1

In einem 2 L-Vierhalskolben, ausgestattet mit Thermometer, Rührer, Wasserauskreiser und Lufteinleitung werden Cyclohexan (240 g), Isosorbid (503 g), MeHQ (1,23 g), hypophosphorige Säure 50%ig (3,08 g) und Cu(II)acetat-Lösung (5%ig, 4,6 g) vorgelegt. Anschließend wird Methacrylsäure zudosiert (755 g, stabilisiert mit 200 ppm MeHQ), Methansulfonsäure (16,8 g) wird hinzugefügt und es wird aufgeheizt. Bei einer Innentemperatur von 84 bis 118°C geht Wasser über. Nach 19 h wird die Reaktion abgebrochen. Die Reaktionsmischung wird nach dem Abkühlen mit Wasser, mit NaOH-Lösung und noch einmal mit Wasser extrahiert. Nach Phasentrennung wird die organische Phase im Vakuum konzentriert. Das Reaktionsgemisch hat folgende Zusammensetzung (GC-FI.-%): Isosorbid 1,1 %, Summe der Isosorbid-Monomethacrylate 22,9 %, Zielprodukt Isosorbiddimethacrylat 75,0 %.

### Vergleichsbeispiel 2

Die Umesterung erfolgt in einem 1,6 L-Doppelmantelreaktor, ausgestattet mit einem AnkerRührer, einer Lufteinleitung, einer Trennkolonne und einem Flüssigkeitsteiler. Das Rücklaufverhältnis beträgt 10:1, später 10:3 (Rücklauf: Ablauf), die Rührgeschwindigkeit 180 U/min und die Lufteinleitung 1,5 L/h. In dieser Apparatur werden 175 g Isosorbid, 0,48 g Methylhydrochinon (MEHQ) und 1200 g Methylmethacrylat (MMA, stabilisiert mit 15 ppm MEHQ) bei Raumtemperatur vorgelegt. 19,1 g Kaliumphosphat werden zugegeben und das Reaktionsgemisch bei einer Badtemperatur von 80°C aufgeheizt, welche im Verlauf der Reaktion auf 100°C angepasst wird. Es wird ein Druck von 400 mbar (abs.) eingestellt und kontinuierlich ein Azeotrop aus Methanol und MMA abdestilliert, wobei die Sumpftemperatur von 75 auf 82°C ansteigt. Nach Beenden der Reaktion wird das Produkt über einen Papierfilter filtriert und das Reaktionsgemisch im Vakuum konzentriert. Das Reaktionsgemisch hat folgende Zusammensetzung (GC-FI%): Isosorbid 0 %, Summe der Isosorbid-Monomethacrylate 4,3 %, Zielprodukt Isosorbiddimethacrylat >85 %.

### Vergleichsbeispiel 3

In einem 4 L-Planschliffreaktor mit aufgesetzter Kolonne (Sulzer CY Packung), Kühler, Flüssigkeitsteiler, Kreuzbalkenrührer, Lufteinleitung sowie einer Vorrichtung zum Waschen der organischen Phase mit anschließendem Phasenseparator und automatisierter Rückführung der organischen Phase werden Heptan (400 g), Methylmethacrylat (2740 g), MeHQ (14,6 g), Dimethylzinndichlorid (52,4 g), 30 %ige Natriummethylatlösung in Methanol (18,32 g) sowie Isosorbid (967 g) vorgelegt und unter Lufteinleitung und Rühren auf eine Sumpftemperatur von 95°C hochgeheizt. Nach Siedebeginn wird ein Rücklaufverhältnis von 6:4 eingestellt. Die Waschwassermenge wird kontinuierlich der Destillatmenge angepasst. Die Sumpftemperatur steigt im Verlauf der Reaktion auf 103°C. In regelmäßigen Abständen werden Sumpfproben gezogen, um den Verlauf der Reaktion zu beobachten. Nach 23 h wird folgende Sumpfzusammensetzung erhalten (GC-FI%): Isosorbid 1,25 %, Summe der Isosorbid-Monomethacrylate 24,3 %, Zielprodukt Isosorbiddimethacrylat 72,7 %.

## Patentansprüche

1. Verfahren zur Herstellung von Isosorbiddi(meth)acrylat durch Umesterung von Alkyl(meth)acrylat mit Isosorbid, umfassend die Schritte:
(i) Reagieren lassen von Alkyl(meth)acrylat mit Isosorbid in Gegenwart eines Titan(IV) oder Zirkonium(IV) enthaltenden Katalysators und eines Stabilisators in Gegenwart eines Schleppmittels, das mit dem in dem Alkyl(meth)acrylat gebundenen Alkohol ein Azeotrop bildet,
(ii) kontinuierliches Abdestillieren des Azeotrops aus Schleppmittel und Alkohol, wobei die Schritte (i) und (ii) gleichzeitig durchgeführt werden, bis Isosorbid im Wesentlichen vollständig umgesetzt ist,
(iii) Zugabe von Wasser zu dem in den Schritten (i) und (ii) erhaltenen, Isosorbid(meth)acrylat enthaltenden Produktgemisch und Abtrennung des Hydrolysats des Titan(IV) oder Zirkonium(IV) enthaltenden Katalysators,
(iv) Abdestillieren von nicht umgesetztem Alkyl(meth)acrylat und Schleppmittel aus dem Produktgemisch,
(v) Abdestillieren von Wasser aus dem Produktgemisch,
wobei Schritt (iv) auch vor Schritt (iii) durchgeführt werden kann und die Schritte (iv) und (v) auch in einem Destillationsschritt durchgeführt werden können.

2. Verfahren nach Anspruch 1, **dadurch gekennzeichnet, dass** das Schleppmittel das Alkyl(meth)acrylat ist.

3. Verfahren nach Anspruch 1, **dadurch gekennzeichnet, dass** das Schleppmittel ein separates, von Alkyl(meth)acrylat verschiedenes Lösungsmittel ist.

4. Verfahren nach Anspruch 3, **dadurch gekennzeichnet, dass** das Schleppmittel ausgewählt ist aus der Gruppe bestehend aus n-Heptan und Cyclohexan.

5. Verfahren nach einem der Ansprüche 1 bis 4, **dadurch gekennzeichnet, dass** die Schritte (iv) und (v) in einem gemeinsamen Destillationsschritt durchgeführt werden.

6. Verfahren nach einem der Ansprüche 1 bis 5, **dadurch gekennzeichnet, dass** das Alkyl(meth)acrylat das Methyl- oder Ethyl(meth)acrylat ist.

7. Verfahren nach einem der Ansprüche 1 bis 6, **dadurch gekennzeichnet, dass** der Katalysator Titan(IV)tetraisopropylat enthält.

8. Verfahren nach einem der Ansprüche 1 bis 7, **dadurch gekennzeichnet, dass** der Stabilisator ausgewählt ist aus Hydrochinonmonomethylether und PTZ.

9. Verfahren nach einem der Ansprüche 1 bis 8, **dadurch gekennzeichnet, dass** aus dem in Schritt (ii) abdestillierten Azeotrop aus Schleppmittel und Alkohol durch Waschen mit Wasser der Alkohol abgetrennt und das Schleppmittel in den Reaktionsbehälter zurückgeführt wird.

10. Verfahren nach einem der Ansprüche 1 bis 9, **dadurch gekennzeichnet, dass** nach Schritt (v) ein Isosorbiddi(meth)acrylat mit einem Gehalt an Nebenkomponenten von < 4 Gew.-% erhalten wird.

11. Verwendung von Isosorbiddi(meth)acrylat als Harzkomponente für Zweikomponenten-Klebemassen.

12. Verwendung nach Anspruch 11, **dadurch gekennzeichnet, dass** die Zweikomponenten-Klebemassen Dübelmassen zur Befestigung von Verankerungsmitteln in Bohrlöchern sind.

13. Verwendung nach Anspruch 11 oder 12, **dadurch gekennzeichnet, dass** das Isosorbiddi(meth)acrylat nach dem Verfahren gemäß einem der Ansprüche 1 bis 10 hergestellt ist.

14. Zweikomponenten-Klebemasse enthaltend
(i) ein Kunstharz mit einer Viskosität bei 23°C zwischen 100 und 10 000 mPa•s, enthaltend Isosorbiddi(meth)acrylat, und
(ii) ein Härtungsmittel für das Kunstharz.

## Claims

1. A process for preparing isosorbide di(meth)acrylate by transesterifying alkyl (meth)acrylate with isosorbide, comprising the steps of:
(i) reacting alkyl (meth)acrylate with isosorbide in the presence of a catalyst comprising titanium(IV) or zirconium(IV) and a stabilizer in the presence of an azeotroping agent which forms an azeotrope with the alcohol bound in the alkyl (meth)acrylate,
(ii) continuously distilling off the azeotrope of azeotroping agent and alcohol,
wherein steps (i) and (ii) are conducted simultaneously until isosorbide has been essentially fully converted,
(iii) adding water to the product mixture which comprises isosorbide (meth)acrylate and is obtained in steps (i) and (ii) and removing the hydrolyzate of the catalyst comprising titanium(IV) or zirconium(IV),
(iv) distilling unconverted alkyl (meth)acrylate and azeotroping agent out of the product mixture,
(v) distilling water out of the product mixture,
wherein step (iv) can also be conducted before step (iii) and steps (iv) and (v) can also be conducted in one distillation step.

2. The process according to claim 1, wherein the azeotroping agent is the alkyl (meth)acrylate.

3. The process according to claim 1, wherein the azeotroping agent is a separate solvent other than alkyl (meth)acrylate.

4. The process according to claim 3, wherein the azeotroping agent is selected from the group consisting of n-heptane and cyclohexane.

5. The process according to any of claims 1 to 4, wherein steps (iv) and (v) are conducted in a combined distillation step.

6. The process according to any of claims 1 to 5, wherein the alkyl (meth)acrylate is methyl or ethyl (meth)acrylate.

7. The process according to any of claims 1 to 6, wherein the catalyst comprises titanium(IV) tetraisopropoxide.

8. The process according to any of claims 1 to 7, wherein the stabilizer is selected from hydroquinone monomethyl ether and PTZ.

9. The process according to any of claims 1 to 8, wherein the alcohol is removed from the azeotrope of azeotroping agent and alcohol distilled off in step (ii) by washing with water and the azeotroping agent is recycled into the reaction vessel.

10. The process according to any of claims 1 to 9, wherein, after step (v), an isosorbide di(meth)acrylate having a content of secondary components of < 4% by weight is obtained.

11. The use of isosorbide di(meth)acrylate as resin component for two-pack adhesive compositions.

12. The use according to claim 11, wherein the two-pack adhesive compositions are plugging compounds for securing anchoring means in drillholes.

13. The use according to claim 11 or 12, wherein the isosorbide di(meth)acrylate has been prepared by the process according to any of claims 1 to 10.

14. A two-pack adhesive composition comprising
(i) a synthetic resin having a viscosity at 23°C between 100 and 10 000 mPa•s, comprising isosorbide di(meth)acrylate, and
(ii) a hardener for the synthetic resin.

## Revendications

1. Procédé de fabrication de di(méth)acrylate d'isosorbide par transestérification de (méth)acrylate d'alkyle avec de l'isosorbide, comprenant les étapes suivantes :
(i) la mise en réaction de (méth)acrylate d'alkyle avec de l'isosorbide en présence d'un catalyseur contenant du titane (IV) ou du zirconium (IV) et d'un stabilisateur en présence d'un agent d'entraînement, qui forme un azéotrope avec l'alcool relié dans le (méth)acrylate d'alkyle,
(ii) l'élimination par distillation continue de l'azéotrope constitué par l'agent d'entraînement et l'alcool, les étapes (i) et (ii) étant réalisées simultanément, jusqu'à ce que l'isosorbide ait réagi essentiellement en totalité,
(iii) l'ajout d'eau au mélange de produits contenant du (méth)acrylate d'isosorbide, obtenu dans les étapes (i) et (ii), et la séparation de l'hydrolysat du catalyseur contenant du titane (IV) ou du zirconium (IV),
(iv) l'élimination par distillation du (méth)acrylate d'alkyle non réagi et de l'agent d'entraînement du mélange de produits,
(v) l'élimination par distillation d'eau du mélange de produits,
l'étape (iv) pouvant également être réalisée avant l'étape (iii), et les étapes (iv) et (v) pouvant également être réalisées en une étape de distillation.

2. Procédé selon la revendication 1, **caractérisé en ce que** l'agent d'entraînement est le (méth)acrylate d'alkyle.

3. Procédé selon la revendication 1, **caractérisé en ce que** l'agent d'entraînement est un solvant séparé, différent du (méth)acrylate d'alkyle.

4. Procédé selon la revendication 3, **caractérisé en ce que** l'agent d'entraînement est choisi dans le groupe constitué par le n-heptane et le cyclohexane.

5. Procédé selon l'une quelconque des revendications 1 à 4, **caractérisé en ce que** les étapes (iv) et (v) sont réalisées en une étape de distillation commune.

6. Procédé selon l'une quelconque des revendications 1 à 5, **caractérisé en ce que** le (méth)acrylate d'alkyle est le (méth)acrylate de méthyle ou d'éthyle.

7. Procédé selon l'une quelconque des revendications 1 à 6, **caractérisé en ce que** le catalyseur contient du tétraisopropylate de titane (IV).

8. Procédé selon l'une quelconque des revendications 1 à 7, **caractérisé en ce que** le stabilisateur est choisi parmi l'éther monométhylique d'hydroquinone et la PTZ.

9. Procédé selon l'une quelconque des revendications 1 à 8, **caractérisé en ce que** l'alcool est séparé de l'azéotrope constitué par l'agent d'entraînement et l'alcool éliminé par distillation dans l'étape (ii) par lavage avec de l'eau, et l'agent d'entraînement est recyclé dans le contenant de réaction.

10. Procédé selon l'une quelconque des revendications 1 à 9, **caractérisé en ce qu'**un di(méth)acrylate d'isosorbide ayant une teneur en composants secondaires < 4 % en poids est obtenu après l'étape (v).

11. Utilisation de di(méth)acrylate d'isosorbide en tant que composant résine pour des matières adhésives bicomposantes.

12. Utilisation selon la revendication 11, **caractérisée en ce que** les matières adhésives bicomposantes sont des matières de chevilles pour la fixation de moyens d'ancrage dans des trous de forage.

13. Utilisation selon la revendication 11 ou 12, **caractérisée en ce que** le di(méth)acrylate d'isosorbide est fabriqué par le procédé selon l'une quelconque des revendications 1 à 10.

14. Matière adhésive bicomposante, contenant :
(i) une résine artificielle ayant une viscosité à 23 °C comprise entre 100 et 10 000 mPa·s, contenant du di(méth)acrylate d'isosorbide, et
(ii) un agent de durcissement pour la résine artificielle.
